# EUROPÄISCHE PATENTSCHRIFT

(11) **EP 2 580 202 B1**
(45) Veröffentlichungstag und Bekanntmachung des Hinweises auf die Patenterteilung: **30.07.2014**
(21) Anmeldenummer: 11723079.7
(22) Anmeldetag: 06.06.2011
(51) Int. Cl.: C07D 309/10

(54) **VERFAHREN ZUR HERSTELLUNG UND ISOLIERUNG VON 2-SUBSTITUIERTEN TETRAHYDROPYRANOLEN**
PROCESS FOR THE PREPARATION AND ISOLATION OF 2-SUBSTITUTED TETRAHYDROPYRANOLS
PROCÉDÉ DE PRODUCTION ET D'ISOLEMENT DE TÉTRAHYDROPYRANOLES SUBSTITUÉS EN 2

(30) Priorität: 10.06.2010 EP 10165455
(43) Veröffentlichungstag der Anmeldung: 17.04.2013
(73) Patentinhaber: BASF SE, 67056 Ludwigshafen (DE)
(72) Erfinder: GRALLA, Gabriele, 68161 Mannheim (DE); BECK, Karl, 76684 Östringen (DE); KLOS, Margarethe, 68309 Mannheim (DE); GRIESBACH, Ulrich, 68305 Mannheim (DE)
(86) Internationale Anmeldenummer: PCT/EP2011/059237
(87) Internationale Veröffentlichungsnummer: WO 2011/154330

(56) Entgegenhaltungen:
- DE-A1- 3 314 395
- JP-A- 2007 154 069
- SU-A1- 825 528

## Beschreibung

Die vorliegende Erfindung betrifft ein Verfahren zur Herstellung und Isolierung von 2-sustituierten 4-Hydroxy-4-methyl-tetrahydropyranolen durch Umsetzung von 3-Methylbut-3-en-1-ol (Isoprenol) mit den entsprechenden Aldehyden in Gegenwart eines stark sauren Kationenaustauschers und anschließender Isolierung bzw. destillativer Abtrennung in einer Trennwandkolonne oder in einer Zusammenschaltung von zwei Destillationskolonnen in Form einer thermischen Kopplung. Speziell betrifft die vorliegende Erfindung ein entsprechendes Verfahren zur Herstellung und Isolierung von 2-Isobutyl-4-hydroxy-4-methyl-tetrahydropyran durch Umsetzung von Isoprenol mit Isovaleraldehyd und anschließender destillativer Abtrennung in einer Trennwandkolonne oder in einer Zusammenschaltung von zwei Destillationskolonnen in Form einer thermischen Kopplung.

In Tetrahedron Letters No. 51, Seiten 4507 - 4508, 1970 wird die Reaktion von 3-Alken-1-olen mit Aldehyden und deren Anwendung zur Herstellung der Aromachemikalien Rosenoxid bzw. Dihydrorosenoxid beschrieben. Dabei wird auch die Umsetzung von 3-Methylbutanal mit Isoprenol unter sauren Bedingungen erwähnt.

In Chemistry of Heterocyclic Compounds, Seiten 1107 - 1109, 1990 wird die Kondensation von Isoprenol mit verschiedenen Aldehyden und Ketonen zu den entsprechenden Di- und Tetrahydropyranen in Gegenwart von Kieselgel oder Al₂O₃ unter lösungsmittelfreien Bedingungen beschrieben. Pyranole werden dabei nur in geringem Ausmaß bei Verwendung von Al₂O₃ erhalten.

Die SU 825 528 offenbart ein Verfahren zur Herstellung von Di- bzw. Tetrahydropyranen und Tetrahydropyranolen durch Umsetzung von 2-Methyl-buten-1-ol-4 (Isoprenol) mit Aldehyden oder Ketonen in Gegenwart eines sauren Katalysators, wobei der saure Katalysator in einer Menge von 0,0001 bis 0,01 Gew.-% bezogen auf die Menge an Isoprenol eingesetzt wird, und die Umsetzung bei einer Temperatur von 0 bis 25°C in einem organischen Lösungsmittel durchgeführt wird. Als Katalysatoren werden der Ionenaustauscherharz KU-2 (sulfoniertes Polystyrolharz), para-Toluolsulfonsäure, Schwefelsäure, Phosphorsäure oder Perchlorsäure genannt. Beispielhaft wird u.a. die Umsetzung von Isoprenol mit Isobutyraldehyd in Gegenwart von KU-2 beschrieben.

EP 1 493 737 A1 offenbart ein Verfahren zur Herstellung von Gemischen von ethylenisch ungesättigten 4-Methyl- bzw. 4-Methylenpyranen und den entsprechenden 4-Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d.h. der Aldehyd im Überschuss eingesetzt wird. Darüber hinaus offenbart das Dokument die anschließende Dehydrierung der genannten Gemische zu den gewünschten ethylenisch ungesättigten Pyranen. Als geeignete Katalysatoren für den ersten Reaktionsschritt werden Mineralsäuren wie Salzsäure oder Schwefelsäure, bevorzugt jedoch Methansulfonsäure oder para-Toluolsulfonsäure genannt.

EP 1 516 879 A1 offenbart ein Verfahren zur Herstellung von ethylenisch ungesättigten 4-Methyl- bzw. 4-Methylenpyranen durch Umsetzung eines entsprechenden Aldehyds mit Isoprenol unter dehydrierenden Bedingungen, wobei die Wassermenge im Reaktor bis zu 0,25 Gew.-% beträgt, während der Umsatz der im Unterschuss eingesetzten Ausgangsverbindung weniger als 50% beträgt. Als hierfür geeignete Katalysatoren werden ebenfalls Mineralsäuren wie Salzsäure oder Schwefelsäure, bevorzugt jedoch Methansulfonsäure oder para-Toluolsulfonsäure genannt.

JP 2007-154069 betrifft 2-substituierte 4-Hydroxy-4-Methyl-tetrahydropyranole mit einem Gehalt des cis-Diastereomeren von 70 bis 95 Gew.-%. Das Dokument offenbart darüber hinaus ein Verfahren zur Herstellung derselben, durch Umsetzung von Isoprenol mit einem entsprechenden Aldehyd in Gegenwart einer wässrigen Lösung eines sauren Katalysators. Dabei muss die Umsetzung bei einer Konzentration der wässrigen Katalysatorlösung entweder im Bereich von 1 bis 10 Gew.-% bei einer Temperatur von 0 bis 100°C durchgeführt werden oder im Bereich von 10 Gew.-% oder darüber bei einer Temperatur von 0 bis 30°C. Als mögliche saure Katalysatoren werden allgemein auch lonentauscherharze genannt.

Es ist bekannt, zur Isolierung von Mehrkomponentensystemen durch Destillation z.B. eine Trennwandkolonne einzusetzen, d.h. eine Destillationskolonne mit zwischen Kopf und Sumpf gelegenem seitlichen Zulauf und einer im Zulaufbereich sich in Längsrichtung der Kolonne erstreckenden Trennvorrichtung zur Verhinderung einer Quervermischung von Brüden und/oder Kondensat. Trennwandkolonnen sind jedoch bislang nicht zur Trennung von stereoisomeren Alkoholen der Formel (I), die üblicherweise eine geringe Siederpunktdifferenz aufweisen, herangezogen worden. Der Erfindung liegt die Aufgabe zu Grunde, ein Verfahren zur Herstellung und Isolierung von 2-substituierten Tetrahydropyranolen anzugeben, mit dem allgemein Produkte mit sehr geringen Siedepunktdifferenzen aus einem Rohgemisch unter geringem Investitions- und Energiebedarf in möglichst reiner Form isoliert werden können.

Destillationskolonnen, die eine Tennwand enthalten, sind an sich bekannt und z. B. beschrieben in der US-A 2,271,134, US-A 4,230,533, der EP-A 122 367, der EP-A 126 288, der EPA 133 510, Chem. Eng. Technol. 10 (1987) 92-98; Chem.-Ing.-Techn. 61 (1989) Nr. 1, 16-25; Gas Separation and Purification 4 (1990) 109 114; Process Engeneering 2 (1993) 33-34; Trans IChemE (1994) Part A 639 644 und Chemical Engineering 7 (1997) 72-76. Die Trennwand kann in die Kolonne fest eingebaut, z. B. eingeschweißt sein, oder aber sie ist lösbar in der Kolonne befestigt, z. B. eingesteckt. Die lösbare Befestigung bietet Vorteile, wie größere Flexibilität, einfachere Packung der Kolonne mit Einbauten und niedrigen Investitionskosten.

Ausgehend von diesem Stand der Technik bestand die Aufgabe der vorliegenden Erfindung in der Bereitstellung eines Verfahrens zur Herstellung und Isolierung von 2-substituierten 4-Hydroxy-4-methyl-tetrahydropyranen, speziell 2-Isobutyl-4-hydroxy-4-methyl-tetrahydropyran, das die gewünschten Verbindungen möglichst
- ausgehend von gut verfügbaren, wohlfeilen Edukten,
- unter Verwendung gut verfügbarer, wohlfeiler Reagenzien
- in verfahrenstechnisch vorteilhafter Weise,
- im technischen Maßstab,
- in hoher Ausbeute,
- in hohem Diastereomerenüberschuss,
- mit möglichst geringer Bildung unerwünschter und zu entsorgender Nebenprodukte,
- in äußerst großer Reinheit und
- mit möglicht vorteilhaften geruchlichen Eigenschaften
zugänglich macht.

Die Aufgabe wurde in überraschender Weise erfindungsgemäß gelöst durch die Bereitstellung eines Verfahrens zur Herstellung von 2-substituierten 4-Hydroxy-4-methyl-tetrahydropyranen der Formel (I) 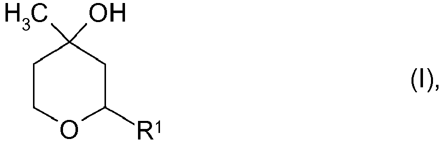 wobei der Rest
- R¹: einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen bedeutet,
umfassend die Umsetzung von 3-Methylbut-3-en-1-ol der Formel (II) 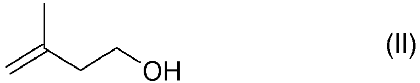 mit einem Aldehyd der Formel (III)

R¹-CHO (III),

wobei der Rest R¹ die gleiche Bedeutung wie in Formel (I) hat und wobei man die Umsetzung in Gegenwart von Wasser und in Gegenwart eines stark sauren Kationenaustauschers durchführt, und anschließend die destillative Abtrennung in einer Trennwandkolonne oder in einer Zusammenschaltung von mindestens zwei Destillationskolonnen in Form einer thermischen Kopplung und einer oder mehrerer Seitenabzugsstellen bei einem absoluten Betriebsdruck bis 500 mbar durchführt.

Für spezielle Anwendungen im Bereich der Aroma- oder Synthesechemie kann es wünschenswert sein, die destillativ abgetrennten Diastereomere in möglichst reiner oder angereicherter Form einsetzen zu können.

Als Ausgangsstoffe zur Durchführung des erfindungsgemäßen Verfahrens dienen 3-Methylbut-3-en-1-ol (Isoprenol) der Formel (II), 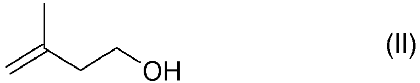 das nach bekannten Verfahren aus Isobuten und Formaldehyd in jedem Maßstab gut zugänglich und kommerziell gut verfügbar ist. An die Reinheit, Qualität oder Herstellverfahren des erfindungsgemäß einzusetzenden Isoprenols sind keine besonderen Anforderungen zu stellen. Es kann in handelsüblicher Qualität und Reinheit mit gutem Erfolg als Ausgangsstoff im Rahmen des erfindungsgemäßen Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew% oder noch mehr bevorzugt 98 bis 99,8 Gew.-%.

Als weiterer Ausgangsstoff zur Durchführung des erfindungsgemäßen Verfahrens dient ein Aldehyd der Formel (III)

R¹-CHO (III),

wobei der Rest R¹ für einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen stehen kann. Dabei ist unter dem Begriff Alkenylrest ein solcher Kohlenwaserstoffrest zu verstehen, der neben Einfachbindungen noch eine oder mehrere, bevorzugt 1 bis 3, besonders bevorzugt 1 oder 2 und ganz besonders bevorzugt eine ethylenische Doppelbindung aufweist.

Unter einem Alkylsubstituenten ist bevorzugt ein solcher zu verstehen der 1 bis 6 Kohlenstoffatome aufweist wie beispielsweise Methyl, Ethyl, n-Propyl, iso-Propyl, n-Butyl, sec-Butyl, tert.-Butyl, n-Pentyl oder n-Hexyl, bevorzugt Methyl, Ethyl, n-Propyl, iso-Propyl, Isobutyl.

Unter einem Alkoxysubstituenten ist bevorzugt ein solcher zu verstehen der 1 bis 6 Kohlenstoffatome, besonders bevorzugt 1 bis 3 Kohlenstoffatome aufweist wie beispielsweise Methoxy, Ethoxy, n-Propyloxy, iso-Propyloxy.

Erfindungsgemäß bevorzugte Aldehyde der Formel (III) sind solche, bei denen der Rest R¹ für einen geradkettigen oder verzweigten Alkyl- oder Alkenylrest mit 1 bis 12 Kohlenstoffatomen, oder einen gegebenenfalls Alkyl- und/oder Alkoxy-substituierten Arylrest mit insgesamt 6 bis 12 Kohlenstoffatomen steht. Erfindungsgemäß ganz besonders bevorzugte Aldehyde der Formel (III) sind solche, bei denen der Rest R¹ für einen geradkettigen oder verzweigten Alkyl- oder Alkenylrest mit 1 bis 12 Kohlenstoffatomen oder bevorzugt mit 1 bis 6 Kohlenstoffatomen, oder für einen Arylrest mit insgesamt 6 Kohlenstoffatomen, d.h. für Phenyl steht. Insbesondere bevorzugte Aldehyde der Formel (III) sind solche, bei denen der Rest R¹ für einen geradkettigen oder verzweigten Alkyl- oder Alkenylrest mit 1 bis 12 Kohlenstoffatomen, ganz besonders bevorzugt mit 1 bis 6 Kohlenstoffatomen steht. Erfindungsgemäß bevorzugte Bedeutungen für den Rest R¹ sind somit beispielsweise Methyl, Ethyl, n-Propyl, iso-Propyl, n-Butyl, iso-Butyl, n-Pentyl, n-Hexyl, n-Heptyl, bevorzugt Methyl, Ethyl, n-Propyl, iso-Propyl, n-Butyl, iso-Butyl, ganz besonders bevorzugt iso-Butyl. Als dementsprechend erfindungsgemäß bevorzugt einzusetzende Aldehyde der Formel (III) seien genannt: Acetaldehyd, Valeraldehyd, Isovaleraldehyd, Pentanal, Hexanal, Heptanal, Benzaldehyd, Citral, Citronellal. Erfindungsgemäß ganz besonders bevorzugt einzusetzende Aldehyde der Formel (III) sind somit Isovaleraldehyd und Benzaldehyd, insbesondere Isovaleraldehyd.

Die vorliegende Erfindung betrifft somit im Rahmen einer bevorzugten Ausführungsform ein Verfahren zur Herstellung und Isolierung von 2-Iso-Butyl-4-Hydroxy-4-methyl-tetrahydropyran der Formel (la) 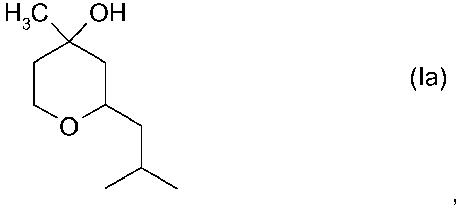 umfassend die Umsetzung von 3-Methylbut-3-en-1-ol der Formel (II) mit Isovaleraldehyd der Formel (IIIa) 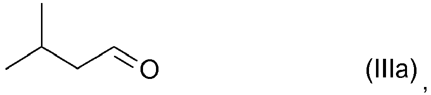 wobei man die Umsetzung in Gegenwart von Wasser und in Gegenwart eines stark sauren Kationenaustauschers durchführt, und anschließend die Isolierung in einer Trennwandkolonne oder in einer Zusammenschaltung von zwei Destillationskolonnen in Form einer thermischen Kopplung und einer oder mehrerer Seitenabzugsstellen bei einem absoluten Betriebsdruck von 3 bis 200 mbar durchführt.

Die vorliegende Erfindung betrifft im Rahmen einer weiteren, ebenfalls bevorzugten Ausführungsform ein Verfahren zur Herstellung und Isolierung von 2-Phenyl-4-hydroxy-4-methyl-tetrahydropyran der Formel (Ib) 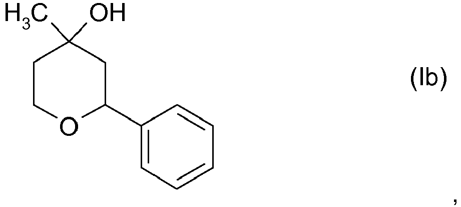 umfassend die Umsetzung von 3-Methylbut-3-en-1-ol der Formel (II) mit Benzaldehyd, wobei man die Umsetzung in Gegenwart von Wasser und in Gegenwart eines stark sauren Kationenaustauschers durchführt, und anschließend die destillative Abtrennung in einer Trennwandkolonne oder in einer Zusammenschaltung von zwei Destillationskolonnen in Form einer thermischen Kopplung und einer oder mehrerer Seitenabzugsstellen bei einem absoluten Betriebsdruck von 3 bis 200 mbar durchführt.

Die im Rahmen des erfindungsgemäßen Verfahrens einzusetzenden Ausgangsstoffe Isoprenol und der jeweils gewählte Aldehyd der Formel (III) können in unterschiedlichen Mengenverhältnissen miteinander umgesetzt werden. So ist es möglich eine der beiden Ausgangsstoffe im Überschuss einzusetzen, wobei die Höhe des gewählten

Überschusses sich in verfahrenstechnisch und wirtschaftlich vorteilhaften Grenzen bewegen sollte, ansonsten im Prinzip jedoch frei gewählt werden kann. Der Stöchiometrie der erfindungsgemäßen Umsetzung von Isoprenol mit dem gewählten Aldehyd der Formel (III) folgend, werden Isoprenol und der Aldehyd der Formel (III), bevorzugt Isovaleraldehyd, in einem molaren Verhältnis im Bereich von 1 zu 2 bis 2 zu 1 eingesetzt, entsprechend einem doppelten molaren Überschuss einer der Ausgangsstoffe. Im Rahmen einer bevorzugten Ausführungsform führt man das erfindungsgemäße Verfahren so durch, dass man Isoprenol und den Aldehyd der Formel (III) in einem molaren Verhältnis von 0,7 zu 1 bis 2 zu 1 einsetzt. Besonders bevorzugt führt man das erfindungsgemäße Verfahren so durch, dass man Isoprenol und den Aldehyd der Formel (III) in einem molaren Verhältnis von 1 zu 1 bis 2 zu 1 einsetzt. Ganz besonders bevorzugt führt man das erfindungsgemäße Verfahren so durch, dass man Isoprenol und den Aldehyd der Formel (III) in einem molaren Verhältnis von 1 zu 1 bis 1,5 zu 1 einsetzt.

Die im Rahmen des erfindungsgemäßen Verfahren zur Herstellung der 2-substituierten 4-Hydroxy-4-methyl-tetrahydropyranen der Formel (I), bevorzugt zur Herstellung von 2-Iso-Butyl-4-Hydroxy-4-methyl-tetrahydropyran der Formel (la) durchzuführende Umsetzung von Isoprenol mit dem gewählten Aldehyd der Formel (III), bevorzugt mit Isovaleraldehyd, wird in Gegenwart von Wasser durchgeführt. Dies bedeutet, dass dem Reaktionsgemisch neben Isoprenol, dem Aldehyd der Formel (III) und dem gewählten stark sauren Kationenaustauscher auch Wasser zugesetzt wird. Zusätzlich kann das Reaktionsgemisch noch geringe Mengen Wasser enthalten, das durch die möglicherweise als unerwünschte Nebenreaktion erfolgende Dehydratisierung des gewünschten Verfahrensproduktes der Formel (I) freigesetzt werden kann.

Üblicherweise führt man die Umsetzung des Isoprenols mit dem gewählten Aldehyd der Formel (III) in Gegenwart von etwa mindestens 10 mol-% Wasser durch, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 10- bis 100-fachem Überschuss oder auch darüber eingesetzt werden. Vorzugsweise bereitet man ein Gemisch aus Isoprenol und dem gewählten Aldehyd der Formel (III), vorzugsweise Isovaleraldehyd, mit der gewählten Menge Wasser, so dass das zugegebene Wasser in dem Gemisch aus Isoprenol und dem gewählten Aldehyd gelöst bleibt d.h. kein zweiphasiges System vorliegt.

Üblicherweise setzt man im Rahmen des erfindungsgemäßen Verfahrens die Ausgangsstoffe Isoprenol und den gewählten Aldehyd der Formel (III) in Gegenwart von mindestens 25 mol-%, bevorzugt von mindestens 50 mol-%, noch mehr bevorzugt von mindestens 75 und noch mehr bevorzugt von mindestens 90 bis etwa 1000 mol-% Wasser um, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

Im Rahmen einer bevorzugten Ausführungsform führt man die erfindungsgemäß durchzuführende Umsetzung so durch, dass man sie in Gegenwart einer mindestens äquimolaren Menge an Wasser durchführt, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht. Demzufolge führt man die erfindungsgemäße Umsetzung von Isoprenol mit dem gewählten Aldehyd der Formel (III) bevorzugt in Gegenwart von 100 bis 250 mol-%, besonders bevorzugt 100 bis 230 mol-%, noch mehr bevorzugt 100 bis 200 mol-% und am meisten bevorzugt in Gegenwart von 100 bis 180 mol-% Wasser durch, wobei sich die Menge an Wasser auf die Menge des gegebenenfalls im Unterschuss eingesetzten Ausgangsstoffes I-soprenol oder den Aldehyd der Formel (III), oder, in dem Fall der äquimolaren Umsetzung der beiden Ausgangsstoffe, auf die Stoffmenge eines der beiden bezieht.

Die genannten Ausgangsstoffe, d.h. Isoprenol und der jeweils gewählte Aldehyd und das in der vorstehenden Menge einzusetzende Wasser können in beliebiger Reihenfolge miteinander in Kontakt gebracht bzw. gemischt werden. Üblicherweise bereitet man ein Gemisch aus Isoprenol und dem gewählten Aldehyd der Formel (III) mit der gewählten Menge Wasser und setzt dieses Gemisch im Rahmen der erfindungsgemäß durchzuführenden Umsetzung ein.

Die im Rahmen des erfindungsgemäßen Verfahrens zur Herstellung der gewünschten 2-substituierten 4-Hydroxy-4-methyl-tetrahydropyranen der Formel (I) durchzuführende Umsetzung von Isoprenol mit dem gewählten Aldehyd der Formel (III) wird in Gegenwart eines stark sauren Kationenaustauschers durchgeführt. Unter dem Begriff stark saurer Kationenaustauscher sind dabei im Rahmen der vorliegenden Erfindung solche Kationenaustauscher in der H⁺-Form zu verstehen, die stark saure Gruppen, in der Regel Sulfonsäuregruppen, aufweisen, deren Matrix gelförmig bzw. makroporös sein kann.

Eine bevorzugte Ausführungsform des erfindungsgemäßen Verfahrens ist dementsprechend dadurch gekennzeichnet, dass man einen stark sauren, Sulfonsäuregruppen aufweisenden bzw. umfassenden Kationenaustauscher einsetzt.

Stark saure Kationenaustauscher sind insbesondere Ionenaustauscherharze in der H(+)-Form. Als solche kommen beispielsweise in Betracht:
- stark saure Ionenaustauscher (wie z.B. Amberlyst, Amberlite, Dowex, Lewatit, Purolite, Serdolit), die auf Polystyrol basieren, und die Copolymere aus Styrol und Divinylbenzol als Trägermatrix mit Sulfonsäuregruppen in H(+)-Form enthalten,
- mit Sulfonsäuregruppen (-SO₃H) funktionalisierte Ionenaustauschergruppen.

Die Ionenaustauscher unterscheiden sich im Aufbau ihrer Polymergerüste, und man unterscheidet gelförmige und makroporöse Harze. Die stark sauren lonentauscherharze werden in der Regel mit Salzsäure und / oder Schwefelsäure regeneriert.

Bei Nafion^{®} handelt es sich um perfluorierte Ionenaustauschmaterialien bestehend aus Fluorkohlenstoff-Basisketten und perfluorierten Seitenketten, die Sulfonsäuregruppen enthalten. Die Harze werden durch eine Copolymerisation von perfluorierten, endständig ungesättigten und mit Sulfonylfluorid funktionalisierten Ethoxylaten mit Perfluorethen hergestellt. Nafion^{®} zählt zu den gelartigen Ionenaustauscherharzen. Als Beispiel für ein solches perfluoriertes polymeres Ionenaustauscherharz sei Nafion^{®} NR-50 genannt.

Eine besonders bevorzugte Ausführungsform des erfindungsgemässen Verfahrens ist dadurch gekennzeichnet, dass man mindestens einen stark sauren Kationenaustauscher in der H(+)-Form einsetzt, wobei der Ionenaustauscher ein Sulfonsäuregruppen aufweisendes Polymergerüst enthält und entweder gelförmig ist oder makroporöse Harze enthält.

Eine ganz besonders bevorzugte Ausführungsform des erfindungsgemässen Verfahrens ist dadurch gekennzeichnet, dass der Ionenaustauscher auf einem Polystyrolgerüst mit Sulfonsäuregruppen oder auf einem perfluorierten Ionenaustauscherharz mit Sulfonsäuregruppen basiert.

Die kommerziell verfügbaren stark sauren Kationenaustauscher sind unter den Handelsnamen Lewatit^{®} (Lanxess), Purolite^{®} (The Purolite Company), Dowex^{®} (Dow Chemical Company), Amberlite^{®} (Rohm and Haas Company), Amberlyst™ (Rohm and Haas Company) bekannt.

Als erfindungsgemäß bevorzugte stark saure Kationenaustauscher seien beispielsweise genannt: Lewatit^{®} K 1221, Lewatit^{®} K 1461, Lewatit^{®} K 2431, Lewatit® K 2620, Lewatit^{®} K 2621, Lewatit^{®} K 2629, Lewatit^{®} K 2649, Amberlite^{®} IR 120, Amberlyst™ 131, Amberlyst™ 15, Amberlyst™ 31, Amberlyst™ 35, Amberlyst™ 36, Amberlyst™ 39, Amberlyst™ 46, Amberlyst™ 70, Purolite® SGC650, Purolite^{®} C100H, Purolite^{®} C150H, Dowex^{®} 50X8, Serdolit^{®} red und Nafion^{®} NR-50.

Im Rahmen einer bevorzugten Ausführungsform führt man die erfindungsgemäß durchzuführende Umsetzung von Isoprenol mit dem gewählten Aldehyd der Formel (III) in Gegenwart mindestens eines stark sauren Kationenaustauschers durch, der ausgewählt ist aus der Gruppe der Kationenaustauscher umfassend Lewatit^{®} K 1221, Lewatit^{®} K 2629, Amberlyst™ 131, Purolite^{®} SGC650, Purolite^{®} C100H, Purolite^{®} C150H, Amberlite^{®} IR 120 und Dowex^{®} 50X8.

Erfindunggsgemäß insbesondere bevorzugte stark saure Kationentauscher sind die Kationenaustauscher Amberlyst™ 131 und/oder Lewatit^{®} K 1221.

Ein erfindungsgemäß ganz besonders bevorzugter stark saurer Kationenaustauscher ist Amberlyst™ 131, der wie die anderen genannten Kationenaustauscher kommerziell verfügbar ist.

Zur Durchführung der erfindungsgemäßen Umsetzung von Isoprenol mit dem Aldehyd der Formel (III) bringt man die genannten Ausgangsstoffe und die gewählte Menge Wasser, vorzugsweise in Form eines Gemisches, mit dem gewählten stark sauren Kationenaustauscher in Kontakt. Die Menge an einzusetzendem Kationenaustauscher ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des gewählten stark sauren Kationenaustauschers durchgeführt werden. Üblicherweise setzt man den gewählten Kationentauscher in einer Menge von etwa 5 bis etwa 40 Gew.-%, bevorzugte in einer Menge von etwa 20 bis etwa 40 Gew.-% und besonders bevorzugt in einer Menge von etwa 20 bis etwa 30 Gew.-%, jeweils bezogen auf die Summe an eingesetztem Isoprenol und Aldehyd der Formel (III), ein. Dabei beziehen sich die Angaben auf den gebrauchsfertigen Kationenaustauscher, der in der Regel mit Wasser vorbehandelt wird und dementsprechend Mengen von bis zu etwa 70 Gew.%, bevorzugt von etwa 30 bis etwa 65 Gew-% und besonders bevorzugt von etwa 40 bis etwa 65 Gew.-% Wasser umfassen kann. Insbesondere bei diskontinuierlicher Verfahrensführung kann sich daher ein darüber hinausgehender Wasserzusatz bei der Durchführung des erfindungsgemäßen Verfahrens erübrigen.

Die genannten stark sauren Kationenaustauscher können sowohl einzeln oder auch in Form von Gemischen untereinander im Rahmen des erfindungsgemäßen Verfahrens eingesetzt werden.

Die erfindungsgemäß durchzuführende Umsetzung kann wahlweise auch in Gegenwart eines unter den Reaktionsbedingungen inerten Lösungsmittels wie beispielsweise tert-Butylmethylether, Cyclohexan, Toluol, Hexan oder Xylol durchgeführt werden. Die genannten Lösungsmittel können alleine oder in Form von Gemischen untereinander eingesetzt werden. Im Rahmen einer bevorzugten Ausführungsform des erfindungsgemäßen Verfahrens führt man die Umsetzung von Isoprenol mit dem gewählten Aldehyd der Formel (III) ohne Zusatz eines organischen Lösungsmittels durch.

Die erfindungsgemäß durchzuführende Umsetzung von Isoprenol mit dem gewählten Aldehyd der Formel (III) in Gegenwart von Wasser und in Gegenwart eines stark sauren Kationentauschers wird üblicherweise bei einer Temperatur im Bereich von 0 bis 60°C, bevorzugt bei einer Temperatur im Bereich von 20 bis 60°C und besonders bevorzugt bei einer Temperatur im Bereich von 20 bis 50°C durchgeführt, wobei sich die Temperatur auf die des Reaktionsgemisches bezieht.

Die erfindungsgemäß durchzuführende Umsetzung kann wahlweise diskontinuierlich oder kontinuierlich durchgeführt werden. Dabei kann man beispielsweise im diskontinuierlichen Fall die Umsetzung so vornehmen, dass man ein Gemisch von Isoprenol, dem gewählten Aldehyd der Formel (III) und Wasser in einem geeigneten Reaktionsgefäß vorlegt und den stark sauren Kationenaustauscher zugibt. Nach Abschluss der Reaktion kann dann der Kationenaustauscher durch geeignete Trennverfahren, vorzugsweise durch Filtration oder auch durch Zentrifugation, vom erhaltenen Reaktionsgemisch abgetrennt werden. Die Reihenfolge des Inkontaktbringens der einzelnen Reaktionskomponenten ist nicht kritisch und kann nach Maßgabe der jeweiligen verfahrenstechnischen Ausgestaltung variiert werden.

Im Rahmen einer bevorzugten Ausführungsform führt man die erfindungsgemäß durchzuführende Umsetzung von Isoprenol mit dem gewählten Aldehyd der Formel (III) kontinuierlich durch. Dazu kann beispielsweise eine Mischung der umzusetzenden Ausgangsstoffe Isoprenol und Aldehyd der Formel (III) mit Wasser bereitet werden und diese Mischung kontinuierlich mit einem stark sauren Kationentauscher in Kontakt gebracht werden. Dazu kann der gewählte Kationenaustauscher beispielsweise in einen geeigneten Durchflussreaktor, beispielsweise einen Rührreaktor mit Zu- und Ablauf oder einen Rohrreaktor eingebracht werden und die Ausgangsstoffe und das Wasser in diesen kontinuierlich ausgetragen werden und das Reaktionsgemisch kontinuierlich ausgetragen werden. Dabei können die Ausgangsstoffe und das Wasser wahlweise als Einzelkomponenten oder auch in Form eines wie vorstehend beschriebenen Gemisches in den Durchflussreaktor eingetragen werden.

Eine bevorzugte Ausführungsform des erfindungsgemäßen Verfahrens betrifft dementsprechend ein kontinuierliches Verfahren zur Herstellung und Isolierung von 2-substituierten 4-Hydroxy-4-methyl-tetrahydropyranen der Formel (I) umfassend die Schritte
a. Bereitstellen eines den gewählten stark sauren Kationenaustauscher enthaltenden Durchflussreaktors;
b. kontinuierliches Eintragen von Isoprenol, dem Aldehyd der Formel (III) sowie Wasser in den Durchflussreaktor;
c. kontinuierliches Inkontaktbringen von Isoprenol, dem Aldehyd der Formel (I-II) sowie Wasser mit dem stark sauren Kationenaustauscher im Durchflussreaktor unter Erhalt eines die gewünschten 2-substituierten 4-Hydroxy-4-methyl-tetrahydropyrane umfassenden Reaktionsgemisches;
d. kontinuierliches Austragen des Reaktionsgemisches aus dem Durchflussreaktor; und
e. Isolierung bzw. destillative Abtrennung in einer Trennwandkolonne oder in einer Zusammenschaltung von zwei Destillationskolonnen in Form einer thermischen Kopplung und einer oder mehrerer Seitenabzugsstellen bei einem absoluten Betriebsdruck bis 500 mbar, vorzugsweise von 3 bis 200 mbar.

Das erfindungsgemäße Verfahren ermöglicht die Herstellung und Isolierung von 2-substituierten 4-Hydroxy-4-methyl-tetrahydropyranen der Formel (I), speziell die Herstellung und Isolierung von 2-Iso-Butyl-4-Hydroxy-4-methyl-tetrahydropyranen der Formel (I). Diese fallen üblicherweise in Form von Reaktionsgemischen an, die neben den gewünschten Zielverbindungen, noch Reste der eingesetzten Ausgangsstoffe, das eingesetzte Wasser sowie möglicherweise in geringem Ausmaß auch die dehydratisierten Nebenprodukte der Formeln (IVa), (IVb) und/oder (IVc) 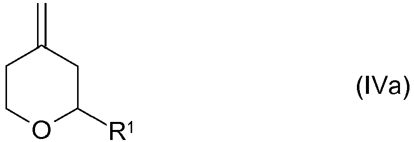 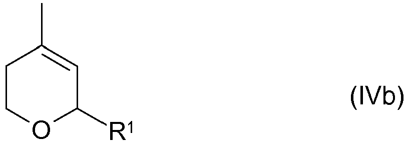 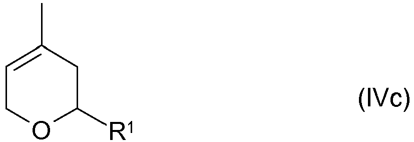 enthalten können. Das erfindungsgemäße Verfahren ermöglicht die Herstellung und Isolierung der gewünschten Hydroxypyrane der Formel (I) bzw. bevorzugt des 2-Iso-butyl-4-hydroxy-4-methyltetrahydropyrans der Formel (la) in hoher Ausbeute und hoher Reinheit, wobei die unerwünschten Dehydratisierungsprodukte der Formeln (IVa) bis (IVc) wenn überhaupt, nur in untergeordnetem Ausmaß anfallen.

Als weitere mögliche Nebenprodukte seien die Acetale der Formel (V) 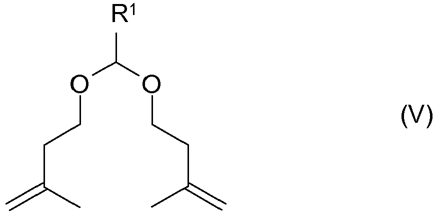 sowie die 1,3-Dioxane der Formel (VI) 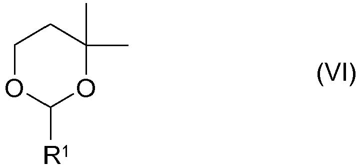 genannt, wobei im erfindungsgemäß bevorzugten Fall der Umsetzung von Isoprenol mit Isovaleraldehyd der Rest R¹ jeweils Iso-Butyl bedeutet (entsprechend den Verbindungen der Formeln (Va) bzw. (VIa)). Diese Nebenprodukte können, genauso wie die nicht umgesetzten bzw. im Überschuss eingesetzten Ausgangsverbindungen in vorteilhafter Weise wieder in die Reaktion zurückgeführt werden.

Die erfindungsgemäß erhaltenen Reaktionsgemische bestehen typischerweise zu etwa 50 bis zu etwa 90 Gew.-%, häufig zu etwa 60 bis zu etwa 80 Gew.-% aus den gewünschten 2-substituierten 4-Hydroxy-4-methyl-tetrahydropyranen der Formel (I) und nur bis zu etwa 20 Gew.-%, bevorzugt nur bis zu etwa 15 Gew.-% und besonders bevorzugt nur bis zu 10 Gew.-% aus den Dehydratisierungsprodukten der Formeln (IVa) bis (IVc), jeweils bezogen auf das Gesamtgewicht des erhaltenen Rohproduktes und im übrigen aus den nicht umgesetzten bzw. im Überschuss eingesetzten Ausgangsstoffen sowie den anderen genannten Nebenprodukten.

Eine bevorzugte Ausführungsform des erfindungsgemäßen Verfahrens betrifft die Herstellung und Isolierung von 2-substituierten 4-Hydroxy-4-methyl-tetrahydropyranen in Form von Gemischen der cis-Diastereomeren der Formel (Ic) 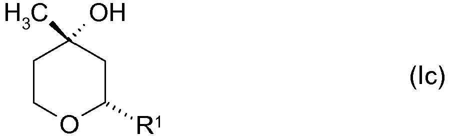 und der trans-Diastereomeren der Formel (Id) 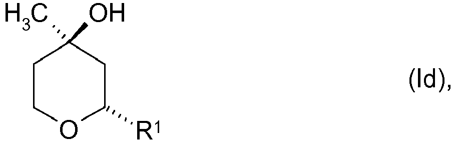 wobei das Diastereomerenverhältnis des cis-Diasteromeren der Formel (Ic) zum trans-Diastereomern der Formel (Id) 65 zu 35 bis 95 zu 5, bevorzugt 70 zu 30 bis 85 zu 15 beträgt, und R¹ die weiter oben angegebene Bedeutung, insbesondere die bevorzugten Bedeutungen, hat.

Insbesondere für die erfindungsgemäß bevorzugte Umsetzung von Isoprenol mit Isovaleraldehyd erhält man im Rahmen des erfindungsgemäßen Verfahrens 2-Isobutyl-4-hydroxy-4-methyl-tetrahydropyran in Form von Gemischen des cis-Diastereomeren der Formel (Ie) 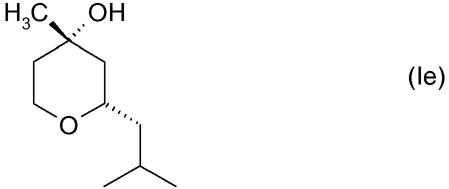 und der trans-Diastereomeren der Formel (If) 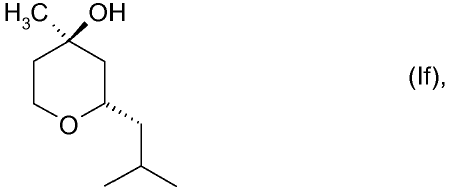 wobei das Diastereomerenverhältnis des cis-Diasteromeren der Formel (Ie) zum trans-Diastereomeren der Formel (If) 65 zu 35 bis 95 zu 5, bevorzugt 70 zu 30 bis 85 zu 15 beträgt. Derartige Gemische eignen sich aufgrund ihrer besonderen geruchlichen Eigenschaften in besonderem Maße zur Verwendung als Aromachemikalien, beispielsweise als Komponente mit Maiglöckchenduft zur Herstellung von Riechstoffkompositionen.

Der Erfindung liegt ferner die Aufgabe zu Grunde, ein Verfahren anzugeben, mit dem stereoisomere Alkohole der Formel (I) aus einem Rohgemisch unter geringem Investitions- und Energiebedarf isoliert werden können.

Erfindungsgemäss wird diese Aufgabe gelöst durch ein Verfahren zur Isolierung zweier stereoisomerer Alkohole der Formel (I) aus einem Rohgemisch durch Rektifikation, wobei man das Rohgemisch seitlich in eine Zulaufkolonne einführt, wenigstens eine mit der Zulaufkolonne gekoppelte Abzugskolonne vorsieht und aus der/den Abzugskolonne(n) einen ersten Alkohol der Formel (I) und einen zweiten Alkohol der Formel (I) abzieht, wobei man die Zulaufkolonne und die Abzugskolonnen so koppelt, dass zumindest im Bereich des Abzugs der Alkohole keine Quervermischung von Brüden und Kondensat zwischen Zulaufkolonne und Abzugskolonne(n) erfolgt.

Die Zulaufkolonne und Ablaufkolonne(n) sind "gekoppelt", d. h. so miteinander verbunden, dass zwischen ihnen ein Stoffaustausch möglich ist. Zur Vermeidung einer Quervermischung von Brüden oder Kondensat zwischen der Zulaufkolonne und der/den Abzugskolonne(n) im Bereich des Abzugs der Alkohole sind die Kolonnen so gekoppelt, dass von der Zulaufstelle zu den Abzugsstellen kein "geradliniger" Weg besteht, sondern jeder Weg von der Zulaufstelle zu den Abzugsstellen einen "aufsteigenden" und einen "absteigenden" Anteil (in beliebiger Reihenfolge) umfasst, d. h. der Austausch von Kondensat und/oder Brüden zwischen diesen Stellen im Wesentlichen nur durch Verdampfung/Rekondensation bzw. Kondensation/Verdampfung erfolgen kann.

Vorzugsweise entnimmt man zumindest einen der Alkohole, insbesondere sowohl den ersten als auch den zweiten Alkohol, an einem Seitenabzug einer Abzugskolonne.

Man geht vorzugsweise so vor, dass man
a) das Rohgemisch in eine Zulaufkolonne mit oberhalb der Zulaufstelle gelegenem Verstärkungsteil und unterhalb der Zulaufstelle gelegenem Abtriebsteil einführt,
b) eine mit dem oberen Ende des Verstärkungsteils kommunizierende obere Vereinigungskolonne mit Kondensator am Kolonnenkopf und eine mit dem unteren Ende des Abtriebsteils kommunizierende untere Vereinigungskolonne mit Aufheizer am Sumpf vorsieht,
c) eine mit der oberen Vereinigungskolonne und der unteren Vereinigungskolonne kommunizierende Abzugskolonne vorsieht, die zwei in der Längsausdehnung der Abzugskolonne beabstandet zueinander angeordnete Seitenabzüge aufweist,
d) den ersten Alkohol am oberen Seitenabzug und den zweiten Alkohol am unteren Seitenabzug abzieht, und
e) am Kopf oder im oberen Bereich der oberen Vereinigungskolonne Leichtsieder und im Sumpf der unteren Vereinigungskolonne Schwersieder abzieht.

Die Zulaufkolonne, Abzugskolonne, obere Vereinigungskolonne und untere Vereinigungskolonne können diskrete Bauelemente sein oder als Abschnitt oder Kammer einer Destillationskolonne ausgebildet sein, die mehrere Funktionen vereint. Der Ausdruck "kommunizierende Kolonnen" bedeutet, dass zwischen ihnen ein Austausch sowohl von aufsteigenden Brüden als auch von ablaufendem Kondensat erfolgt.

In einer bevorzugten Ausführungsform verwendet man zur Destillation eine so genannte Trennwandkolonne, das heißt die Zulaufkolonne und die Abzugskolonne sind als beidseitig zu je einem Vereinigungsraum offene Teilkammern ausgebildet, die sich über einen Abschnitt der Längsausdehnung einer Destillationskolonne erstrecken und durch eine Trennwand voneinander getrennt sind. Die Trennwand kann in die Kolonne fest eingebaut, z. B. eingeschweißt sein, oder aber sie ist lösbar in der Kolonne befestigt, z. B. eingesteckt. Die lösbare Befestigung bietet Vorteile, wie größere Flexibilität, einfachere Packung der Kolonne mit Einbauten und niedrigen Investitionskosten.

In alternativen Ausführungsformen verwendet man zur Destillation thermisch gekoppelte Kolonnen, z. B. eine Destillationskolonne mit einer thermisch gekoppelten Vorkolonne oder eine Destillationskolonne mit einer thermisch gekoppelten Nachkolonne. Destillationskolonnen mit beigeschalteten Hilfskolonnen sind an sich bekannt und dem Fachmann geläufig.

Bei den stereoisomeren Alkoholen handelt es sich in dem erfindungsgemässen Verfahren um die Alkohle der Formel (I), insbesondere solche der Formeln (Ic) und (Id) bzw. ganz besonders bevorzugt solche der Formeln (Ie) und (If). In einer besonders bevorzugten Ausführungsform handelt es sich bei dem ersten Alkohol um Verbindungen der Formel (Ie), bei dem zweiten um die Verbindung der Formel (If). Ein entsprechendes Rohgemisch kann beispielsweise bei der oben angegebenen Synthese von in 2-Stellung substituierten Tetrahydropyranolen der Formel (I) durch Umsetzung der Komponenten (II) und (III) in Gegenwart eines stark sauren Ionenaustauschers hergestellt werden.

Das Rohgemisch enthält typischerweise 15 bis 20 Gew.-% des trans-Pyranols der Formel (If); 45 bis 65 Gew.-% des cis-Pyranols der Formel (Ie); 10 bis 20 Gew.-% niedriger als der erste Alkohol siedende Verbindungen; 1 bis 3 Gew.-% höher als der zweite Alkohol siedende Verbindungen; das Rohgemisch ist vorzugsweise im Wesentlichen frei von Verbindungen, die zwischen dem ersten und zweiten Alkohol sieden.

Das erfindungsgemässe Verfahren wird im Folgenden unter Bezug auf die bevorzugte Ausgestaltung unter Verwendung einer Trennwandkolonne mit zwei flüssigen Seitenabzügen näher erläutert. Sämtliche Ausführungen gelten entsprechend für alternative Kolonnenanordnungen, wie thermisch gekoppelte Kolonnen.

Der Kopfdruck der Kolonne, an deren Kopf die niedriger als der erste Alkohol siedenden Verbindungen abgezogen werden, insbesondere der oberen Vereinigungskolonne, beträgt vorzugsweise bis 400 mbar, vorzugsweise 3 bis 200 mbar, besonders bevorzugt 3 bis 100 mbar, und ganz besonders bevorzugt 4 bis 60 mbar.

Eine bevorzugte Ausführungsform des erfindungsgemässen Verfahrens ist dadurch gekennzeichnet, dass die Zulaufkolonne und die Abzugskolonne als beidseitig zu je einem Vereinigungsraum offene Teilkammer ausgebildet sind, die sich über einen Abschnitt der Längsausdehnung einer Destillationskolonne erstrecken, und durch eine Trennwand voneinander getrennt sind.

Die Summe der theoretischen Trennstufen von oberer Vereinigungskolonne, Zulaufkolonne, Abzugskolonne und unterer Vereinigungskolonne beträgt vorzugsweise 30 bis 110, insbesondere 40 bis 90.

Vorzugsweise entfallen auf die obere Vereinigungskolonne 5 bis 50%, insbesondere 15 bis 30%, auf den Verstärkungsteil der Zulaufkolonne 5 bis 50%, insbesondere 15 bis 30%, auf den Abtriebsteil der Zulaufkolonne 5 bis 50%, insbesondere 15 bis 40%, auf den oberhalb des oberen Seitenabzugs gelegenen Teil der Abzugskolonne 5 bis 50%, insbesondere 15 bis 30%, auf den zwischen den Seitenabzügen gelegenen Teil der Abzugskolonne 5 bis 50%, insbesondere 15 bis 30%, auf den unterhalb des unteren Seitenabzugs gelegenen Teil der Abzugskolonne 5 bis 50%, insbesondere 15 bis 40%, und auf die untere Vereinigungskolonne 5 bis 50%, insbesondere 15 bis 30%, der Summe der theoretischen Trennstufen von oberer Vereinigungskolonne, Zulaufkolonne, Abzugskolonne und unterer Vereinigungskolonne.

Vorzugsweise beträgt das Verhältnis der Summe der theoretischen Trennstufen der Zulaufkolonne zur Summe der theoretischen Trennstufen der Abzugskolonne 0,8 bis 1,1, insbesondere 0,9 bis 1,0.

Die Zulaufkolonne, die obere Vereinigungskolonne, die untere Vereinigungskolonne und die Abzugskolonne enthalten vorzugsweise trennwirksame Einbauten, wie Trennböden, geeordnete Packungen, z. B. Blech- oder Gewebepackungen wie Sulzer Meltallapak, Sulzer BX, Montz B1 oder Montz A3 oder Kühni Rhombopak, oder regellose Schüttungen von Füllkörpern, wie z. B. Dixon-Ringen, Raschig-Ringen, High-Flow-Ringen oder Raschig-Super-Ringen. Es ist bevorzugt, dass zumindest die Zulaufkolonne und/oder Abzugskolonne ganz oder in Teilbereichen mit geordneten Packungen versehen sind. Besonders bewährt haben sich geordnete Packungen, vorzugsweise Blech- oder Gewebepackungen, einer spezifischen Oberfläche von 100 bis 750 m²/m³, insbesondere 250 bis 500 m²/m³. Sie gestalten hohe Trennleistungen bei niedrigen Druckverlusten.

Eine bevorzugte Ausführungsform des erfindungsgemässen Verfahrens ist dadurch gekennzeichnet, dass die Zulaufkolonne und/oder Abzugskolonne ganz oder in Teilbereichen mit geordneten Packungen oder Füllkörpern versehen sind.

Bei Einsatz einer Trennwandkolonne hat es sich als günstig erwiesen, wenn die Trennwand zumindest in einem Teilbereich wärmeisolierend ist, z. B. doppelwandig mit dazwischenliegendem Gasraum, ausgefüllt ist. Eine Beschreibung der verschiedenen Möglichkeiten der thermischen Isolierung der Trennwand findet sich in der EP-A 640 367.

Das Rohgemisch wird vorzugsweise mengengeregelt in die Zulaufkolonne eingeführt, beispielsweise in dem es mittels einer steuerbaren Pumpe gefördert oder über eine ausreichende statische Zulaufhöhe von z. B. wenigstens einem Meter über ein steuerbares Ventil zugeführt wird. Zweckmäßigerweise sieht man eine Mindestzulaufmenge vor, die nicht unterschritten werden darf und die z. B. 30% unter dem Normalwert liegt, für den die Anlage ausgelegt ist.

Es kann bisweilen vorteilhaft sein, das Rohgemisch vor zu verdampfen und teilweise oder vollständig dampfförmig in die Zulaufkolonne einzuführen. Das teilweise vorverdampfte Rohgemisch wird dann als zweiphasiger Strom oder in Form von eines flüssigen und eines dampfförmigen Stroms in die Zulaufkolonne eingeführt. Die Vorverdampfung bietet sich an, wenn das Rohgemisch größere Mengen an Leichtsiedern enthält. Durch die Vorverdampfung kann der Abtriebsteil der Zulaufkolonne entlastet werden.

Es ist bevorzugt, die Seitenabzüge in flüssiger Form zu entnehmen. Der Abzug des ersten Alkohols wird vorzugsweise über Messwerte der Temperatur an einer zwischen dem oberen Ende der Abzugskolonne und oberem Seitenabzug gelegenen Stelle der Abzugskolonne geregelt, die vorzugsweise 3 bis 8, insbesondere 4 bis 6 theoretische Trennstufen unterhalb des oberen Endes der Abzugskolonne liegt. Die abgezogene Menge wird mit steigender Temperatur erhöht und umgekehrt. Zweckmäßigerweise begrenzt man den Abzug so, dass die Menge an Kondensat, die auf den zwischen den Seitenabzügen gelegenen Teil der Abzugskolonne läuft, nicht unter einen Mindestwert sinkt, der nicht unterschritten werden darf und der z. B. 30% unter des Normalwerts liegt, für den die Anlage ausgelegt ist.

Eine bevorzugte Ausführungsform des erfindungsgemässen Verfahrens ist dadurch gekennzeichnet, dass man den ersten Alkohol und den zweiten Alkohol in flüssiger Form an den Seitenabzügen der Trennwandkolonne abzieht.

Der Abzug des zweiten Alkohols wird vorzugsweise über Messwerte für den Füssigkeitsstand im Aufheizer geregelt. Die abgezogene Menge wird mit steigendem Flüssigkeitsstand erhöht und umgekehrt.

Das Rücklaufverhältnis des Kondensators wird vorzugsweise über Messwerte für die Temperatur an einer Stelle im Bereich der oberen Vereinigungskolonne, die vorzugsweise 3 bis 8, insbesondere 4 bis 6, theoretische Trennstufen unterhalb des oberen Endes angeordnet ist, geregelt. Selbstverständlich kann anstelle des Rücklaufverhältnisses auch die Destillat-Entnahmemenge, d.h. der Abzug der Leichtsieder, geregelt werden, wodurch mittelbar das Rücklaufverhältnis geregelt wird.

Die Entnahme von Schwersiedern aus dem Sumpf wird vorzugsweise über Messwerte für die Temperatur an einer Stelle im Bereich der unteren Vereinigungskolonne, die vorzugsweise 3 bis 8, insbesondere 4 bis 6, theoretische Trennstufen oberhalb des unteren Endes gelegen ist, geregelt.

Die Heizleistung des Aufheizers wird geeigneterweise über den Differenzdruck zwischen einer Stelle am oberen Ende der oberen Vereinigungskolonne und einer Stelle am unteren Ende der unteren Vereinigungskolonne geregelt.

Der Brüdenstrom aus der unteren Vereinigungskolonne wird auf die Zulaufkolonne und die Abzugskolonne vorzugsweise in einem Verhältnis von 0,8 bis 1,2, insbesondere 0,9 bis 1, 1, aufgeteilt. Die Einstellung eines bestimmten Aufteilungsverhältnisses gelingt z. B. durch Veränderung des relativen Querschnittes der Zulauf- und Abzugskolonne, durch Auswahl und/oder Dimensionierung der trennwirksamen Einbauten und/oder den Einbau einen Druckverlust erzeugender Einrichtungen, wie Blenden.

Der Kondensatstrom aus der oberen Vereinigungskolonne wird auf die Zulaufkolonne und die Abzugskolonne vorzugsweise in einem Verhältnis von 0,1 bis 1,0, insbesondere 0,3 bis 0,6, aufgeteilt.

Zur Entnahme oder Aufteilung von Kondensat an einer Stelle einer Kolonne, z.B. zur Aufteilung des Kondensats aus der oberen Vereinigungskolonne auf die Zulauf- und Abzugskolonne oder zur Entnahme von flüssigen Seitenabzügen, wird das Kondensat geeigneterweise zu innerhalb oder außerhalb der Kolonne angeordneten Sammelbehältern geleitet. Die Sammelbehälter dienen als Pumpenvorlage oder sorgen für eine ausreichend hohe statische Flüssigkeitshöhe, die eine durch Stellorgane, beispielsweise Ventile, geregelte Flüssigkeitsentnahme oder -aufteilung ermöglicht. Bei Verwendung gepackter Kolonnen sind zweckmäßigerweise Fangböden vorgesehen, von denen das Kondensat zu den Sammelbehältern geleitet wird.

Bei der destillativen Reingewinnung der Alkohole der Formeln (Ie) und (If) aus Rohgemischen soll die gewonnene Fraktion neben der gewünschten Verbindung einen möglichst geringen Anteil an höher oder niedriger siedenden Verunreinigungen enthalten. Je nach beabsichtigter Verwendung sind die Spezifikationen hinsichtlich des maximal zulässigen Gehalts höher oder niedriger siedender Verbindungen unterschiedlich. In der Regel werden einzelne für das Trennproblem kritische Komponenten, meist solche mit einer engen Siedepunktsdifferenz zur angestrebten Verbindung oder solche, deren Anwesenheit auch in geringen Konzentrationen besonders störend ist, sogenannte Schlüsselverbindungen, oder die Summe von mehreren Schlüsselverbindungen spezifiziert.

Die Einhaltung der Spezifikation für die Konzentration an höher als der erste Alkohol siedenden Verbindungen (einschließlich des zweiten Alkohols) in der ersten Alkohol-reinfraktion wird im erfindungsgemässen Verfahren vorzugsweise durch das Aufteilungsverhältnis des Kondensatstroms aus der oberen Vereinigungskolonne geregelt. Bei Verwendung einer Trennwandkolonne ist dies das Aufteilungsverhältnis der Flüssigkeit am oberen Ende der Trennwand. Hierzu gewinnt man im Bereich der oberen Vereinigungskolonne, vorzugsweise an deren unterem Ende, Messwerte für die Konzentration wenigstens einer höher als der erste Alkohol siedenden Verbindung und bildet aus diesen Messwerten Regelungseingriffe für das Aufteilungsverhältnis des Kondensatstroms aus der oberen Vereinigungskolonne in die Zulaufkolonne und die Abzugskolonne. Mit steigender Konzentrationen an höher siedenden Verbindungen wird ein zunehmend größerer Anteil des Kondensats in die Zulaufkolonne geleitet. Die Konzentration an höher als der erste Alkohol siedenden Verbindungen sollte am unteren Ende der oberen Vereinigungskolonne 10 bis 80%, vorzugsweise 30 bis 50%, des maximal zulässigen Wertes der Konzentration dieser Verbindungen in der ersten Alkohol-Reinfraktion betragen.

Die Einhaltung der Spezifikation für die Konzentration an niedriger als der zweite Alkohol siedenden Verbindungen (einschließlich des ersten Alkohols) in der zweiten Alkoholreinfraktion wird im erfindungsgemässen Verfahren vorzugsweise über die Heizleistung des Aufheizers geregelt. Hierzu gewinnt man im Bereich der unteren Vereinigungskolonne, vorzugsweise an deren oberem Ende, Messwerte für die Konzentration wenigstens einer niedriger als der zweite Alkohol siedenden Verbindung und bildet aus diesen Messwerten Regelungseingriffe für die Heizleistung des Aufheizers. Mit steigender Konzentration an niedriger siedenden Verbindungen wird die Heizleistung erhöht und umgekehrt. Die Konzentration an niedriger als der zweite Alkohol siedenden Verbindungen sollte am oberen Ende der unteren Vereinigungskolonne 10 bis 80%, vorzugsweise 30 bis 50%, des maximal zulässigen Wertes der Konzentration dieser Verbindungen in der zweiten Alkohol-Reinfraktion betragen.

Um Messwerte für die Konzentration der vorstehend angesprochenen Schlüsselverbindungen zu gewinnen, kann man an der jeweiligen Stelle kontinuierlich oder periodisch gasförmige oder flüssige Proben entnehmen und hinsichtlich ihrer Zusammensetzung untersuchen, vorzugsweise gaschromatographisch. Zur Probenentnahme sind vorzugsweise geeignete Probenentnahmestellen in den Destillationskolonnen vorgesehen, über die Lanzen in die Kolonnen eingeführt werden können. In vielen Fällen kann eine ausreichende Aussage über die Zusammensetzung der Brüden bzw. des Kondensats an einer Stelle einer Destillationskolonne auch anhand einer einfachen Temperaturmessung getroffen werden, wenn die Korrelation des Temperaturprofils mit der Gemischzusammensetzung bekannt oder zuvor bestimmt worden ist.

Bei der Destillation von Riechstoffen in aufeinanderfolgenden Kolonnen, in denen die Komponenten in der Reihenfolge ihrer Flüchtigkeit über Kopf abgenommen werden, kann die thermische Belastung aufgrund der hohen Verweilzeiten in den Kolonnensümpfen zu einer Produktverschlechterung und/oder zur Bildung unerwünschter Geruchsträger führen. Das kann dazu führen, dass das erhaltene Reinprodukt außerhalb der geforderten Spezifikation liegt und/ oder die olfaktorische Prüfung nicht passiert. Das erfindungsgemässe Verfahren minimiert die thermische Belastung des zu trennenden Gemisches und umgeht die damit verbundenen Nachteile.

Die verschiedenen Ausführungsformen von thermisch gekoppelten Destillationskolonnen bzw. von Trennwandkolonnen sind in der Literatur bekannt, z.B. DE-A-102 23 971.

Die folgenden Beispiele dienen der Erläuterung der Erfindung, ohne sie in irgendeiner Weise zu beschränken:
Gaschromatographische Analysen wurden nach der folgenden Methode durchgeführt: 30 m DB-WAX, ID.: 0,32 mm, FD.: 0,25 µm; 50°C, 3°C/min - 170 °C, 20°C/min auf 230 °C - 17 min; Inj. 200°C, Det. 280°C, t_{R} = min; t_{R} (Isovaleraldehyd): 4,1; t_{R} (Dihydropyran-Isomere der Formeln (IVa) bis (IVc)): 10,0; 11,8; 12,3; t_{R} (Isoprenol): 10,6; t_{R} (1,3-Dioxan (Va)): 12,1; t_{R} (Acetal (Via)): 24,1; t_{R} (trans-Pyranol der Formel (Ie)): 28,2; t_{R} (cis-Pyranol der Formel (Id)): 29,8. Konzentrationen der erhaltenen Rohprodukte (Gew.-%) wurden GC-analytisch mittels internem Standard ermittelt.

Der Wassergehalt der erhaltenen Rohprodukte wurde mittels Karl-Fischer-Titration bestimmt.

### Beispiel 1:

Eine Apparatur bestehend aus einem Doppelmantelglasrohrreaktor mit einem Innendurchmesser von 2 cm und einer Länge von 36 cm wurde mit 50 g des stark sauren Kationenaustauschers Amberlyst™ 131 gefüllt. Der Kationenaustauscher wurde vor dem Einsatz zunächst mehrmals mit Wasser gewaschen, dann einmal mit Methanol und abschließend mit Wasser Methanol-frei gewaschen.

Der Doppelmantelglasreaktor wurde mit einem Gemisch aus Isovaleraldehyd (112,5 g, 1.31 mol), Isoprenol (125 g, 1.45 mol) und 12,5 g Wasser bei Raumtemperatur befüllt. Die Reaktionslösung wurde 4 h bei einer Temperatur von 35 °C mit einem Umpumpvolumen von 490 ml/h umgepumpt. Anschließend wurde die Reaktionslösung weitere 10 h bei einer Temperatur von 40 °C mit einem Umpumpvolumen von 490 ml/h umgepumpt. Der Doppelmantelglasreaktor wurde mit einer Temperatur von 35-40°C betrieben. Man erhielt ein Rohprodukt in einer Menge von 247,2 g (Ausbeute bzgl. cis/trans-Pyranol (Ie und If) 74%) mit der folgenden Zusammensetzung:

| | |
|---|---|
| Isovaleraldehyd: | 0,53 GC-Gew.%, |
| Isoprenol: | 1,23 GC-Gew.%, |
| Dihydropyran-Isomere (IVa - c): | 8,96 GC-Gew.% |
| 1,3-Dioxan (Va): | 8,84 GC-Gew.%, |
| Acetal (VIa): | 0,49 GC-Gew.%, |
| trans-Pyranol (If): | 18,34 GC-Gew.%, |
| cis-Pyranol (Ie): | 49,27 GC-Gew.%. |
| Wasser: | 6,0% |

### Beispiel 2:

Der Doppelmantelglasreaktor wurde mit einem Gemisch aus Isovaleraldehyd (77.4 g, 0.9 mol), Isoprenol (86.1 g, 1.0 mol) und 8.6 g Wasser bei Raumtemperatur befüllt. Die Reaktionslösung wurde 10 h bei einer Temperatur von 25 °C mit einem Umpumpvolumen von 1.5 l/h umgepumpt. Der Doppelmantelglasreaktor wurde auf 25°C temperiert. Man erhielt ein Rohprodukt in einer Menge von 169.4 g (Ausbeute bzgl. cis/trans-Pyranol (Ie und If) 79%) mit der folgenden Zusammensetzung:

| | |
|---|---|
| Isovaleraldehyd: | 0,44 GC-FI.%, |
| Isoprenol: | 3,57 GC-FI.%, |
| Dihydropyran-Isomere (IVa - c): | 9,76 GC-FI.%, |
| 1,3-Dioxan (Va): | 3,16 GC-FI.%, |
| Acetal (VIa): | 0,99 GC-FI.%, |
| trans-Pyranol (If): | 18,91 GC-Gew.%, |
| cis-Pyranol (Ie): | 54,13 GC-Gew.%. |
| Wasser | 6,9% |

### Beispiel 3: Destillation mittels zweier Laborkolonnen

Die erste Kolonne war aus zwei doppelmanteligen, innenverspiegelten und evakuierten Glasschüssen (oben 800 mm lang; unten 640 mm) mit 43 mm Innendurchmesser aufgebaut. Bei den Einbauten handelte es sich um Montz A3-750 Metall-GewebePackungen. Die Trennwirkung der Kolonne entspricht etwa 15 theoretischen Böden. Die Beheizung der Kolonne erfolgte mit einem ölbeheizten Laborumlaufdünnschichtverdampfer. Am Kopf der Kolonne wurden die Brüden mit einem kühlwassergekühlten Glaskondensator niedergeschlagen. Für die Trennung der beiden Phasen des Kopfkondensates war ein Glas-Phasenscheider eingebaut. Die untere wässrige Phase wurde standgeregelt herausgefahren. Die obere organische Phase wurde mithilfe eines Rücklaufteilers in einem festen Verhältnis aufgeteilt. Ein Teil wurde als Kopfprodukt abgefahren. Der andere Teil wurde oben auf die Gewebepackung aufgegeben. Temperaturen an verschiedenen Höhen in der Kolonne sowie der Kopfdruck und der Druckverlust über die Kolonne wurden mittels eines Messwerterfassungssystems gemessen. Die Kolonne verfügte über Durchflussmessungen in den Zu- und Abläufen sowie eine Mengenmessung des Rücklaufs, die als Regelgröße für die Vorlauftemperatur des Ölthermostaten diente. Durch diese Regelung wurde eine konstante Rücklaufmenge gewährleistet, wodurch sich auch ein konstanter Differenzdruck einstellte. Der Zulauf zur Kolonne erfolgte zwischen den beiden Kolonnenschüssen. Der Zulaufstrom wurde mit Raumtemperatur zugefahren. Der Mengenstrom betrug 995 g/h. Das Gemisch hatte folgende Zusammensetzung:
7,3 % Wasser (Karl-Fischer-Methode)
0,79 GC-Gew-% Isovaleraldehyd
3,2 GC-Gew-% Isoprenol
7,4 GC-Gew-% Dihydropyran-Isomere (IVa - c)
6,0 GC-Gew-% 1,3 Dioxan (Va)
0,36 GC-Gew-% Acetal (Via)
18,0 GC-Gew-% trans-Pyranol (If)
52,3 GC-Gew-% cis-Pyranol (Ie)

Die Kolonne wurde bei 50 mbar Kopfdruck und einer Rücklaufmenge von 270 g/h betrieben. Dabei stellte sich ein Druckverlust von ca. 2,5 mbar ein. Am Kopf der Kolonne wurde eine Temperatur von 62 °C und im Sumpf eine Temperatur von 130 °C gemessen. Die Sumpfabzugsmenge wurde über eine Waagensteuerung fest auf 785 g/h eingestellt. Die Kopfabzugsmenge betrug 130 g/h.
Gaschromatographische Analysen wurden nach der folgenden Methode durchgeführt: 30 m DB-WAX, ID.: 0,32 mm, FD.: 0,25 µm; 50°C, 3°C/min - 170 °C, 20 °C/min auf 230 °C - 17 min; Inj. 200°C, Det. 280°C, t_{R} = min; t_{R} (Isovaleraldehyd): 4,1; t_{R} (Dihydropyran-Isomere der Formeln (IVa) bis (IVc)): 10,0; 11,8; 12,3; t_{R} (Isoprenol): 10,6; t_{R} (1,3-Dioxan (Va)): 12,1; t_{R} (Acetal (Via)): 24,1; t_{R} (trans-Pyranol der Formel (If)): 28,2; t_{R} (cis-Pyranol der Formel (Ie)): 29,8. Konzentrationen der erhaltenen Rohprodukte (Gew. %) wurden GC-analytisch mittels internem Standard ermittelt. Der aus dem Phasenscheider am Kopf der Kolonne abgezogene Kopfstrom enthielt:
2,5 % Wasser (Karl-Fischer-Methode)
4,6 GC-Flächen-% Isovaleraldehyd
40,5 GC-Flächen-% Dihydropyran-Isomere (IVa - c)
23,9 GC-Flächen-% Isoprenol
28,1 GC-Flächen-% 1,3 Dioxan (Va)

Im Sumpfabzug wurden
0,03 % Wasser (Karl-Fischer-Methode) und durch GC-Analyse
3,1 GC-Gew-% Dihydropyran-Isomere (IVa - c)
2,6 GC-Gew-% 1,3 Dioxan (Va)
22,4 GC-Gew-% trans-Pyranol (If)
65,6 GC-Gew-% cis-Pyranol (Ie) bestimmt.

Die Destillationsausbeute bezüglich cis- und trans-Pyranol (Ie und If) betrug 100%.

Die zweite Laborkolonne wurde als Trennwandkolonne aufgebaut. Sie war aus drei doppelmanteligen, innenverspiegelten und evakuierten Glasschüssen mit 43 mm Innendurchmesser aufgebaut. Der mittlere Kolonnenschuss mit einer Gesamtlänge von 105 cm wurde mit einer fest eingeschmolzenen Trennwand aus etwa 1 mm starkem Glas versehen. Im Bereich der Trennwand ist die Kolonne mit 1 m Montz A3 1000-Packung auf der Zulaufseite und 0,9 m Montz A3 1000-Packung auf der Abnahmeseite ausgerüstet. Oberhalb und unterhalb der Trennwand wurden 50 cm lange Glassschüsse eingesetzt, die je mit 33 cm Sulzer DX- Packungen ausgerüstet wurden. Die Trennleistung im Trennwandbereich betrug ca. 32 theoretische Stufen. Die Gesamtzahl der theoretischen Stufen inklusive des Trennwandbereiches betrug ca. 50.
Die Beheizung der Kolonne erfolgte mit einem ölbeheizten Laborumlaufdünnschichtverdampfer. Am Kopf der Kolonne wurden die Brüden mit einem kühlwassergekühlten Glaskondensator niedergeschlagen.
Temperaturen an verschiedenen Höhen in der Kolonne sowie der Kopfdruck und der Druckverlust über die Kolonne wurden mittels eines Messwerterfassungssystems gemessen. Die Kolonne verfügte über Durchflussmessungen in den Zu- und Abläufen sowie eine Mengenmessung des Rücklaufs, die als Regelgröße für die Vorlauftemperatur des Ölthermostaten diente. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.
Der Zulauf wurde auf Höhe der Mitte des Trennwandteiles zugegeben. Als Zulaufstrom wurde das Gemisch aus dem Sumpfablauf der ersten Kolonne verwendet. Der Zulaufmengenstrom betrug 300 g/h.

Die Kolonne wurde bei 10 mbar Kopfdruck und einem Rücklauf von 400 g/h betrieben. Dabei stellte sich ein Druckverlust von etwa 1,5 mbar ein. Am Kopf der Kolonne wurde eine Temperatur von 78°C und im Sumpf eine Temperatur von 127 °C (± 0,5 K) gemessen. Der Sumpfabzug wurde über eine Waagensteuerung fest auf 14 g/h (± 1 g/h) und die Destillatabnahme auf 26 g/h (± 1 g/h) eingestellt. Das Rücklaufverhältnis betrug somit etwa 15:1.
Die Flüssigkeit wurde oberhalb der Trennwand in einem Verhältnis von 1 : 2 (Zulauf- : Entnahmeteil) aufgeteilt. Auf der der Zugabeseite gegenüberliegenden Seite der Trennwand wurde auf gleicher Höhe wie der Zulaufstrom ein flüssiger Seitenabzug entnommen. Der Mengenstrom war fest auf 260 g/h eingestellt.
Das am Seitenabzug gewonnene Reinprodukt enthielt
23,3 GC-Flächen-% trans-Pyranol (If)
76,5 GC-Flächen-% cis-Pyranol (Ie)

Der am Kopf der Kolonne abgezogene Kopfstrom enthielt:
0,25 GC-Gew-% Isovaleraldehyd
34,9 GC-Gew-% Dihydropyran-Isomere (IVa - c)
0,54 GC-Gew-% Isoprenol
28,5 GC-Gew-% 1,3 Dioxan(Va)
24,6 GC-Gew-% trans-Pyranol (If)
2,1 GC-Gew-% cis-Pyranol (Ie)

Der am Sumpf der Kolonne abgezogene Strom enthielt:
0,45 GC-Gew-% trans-Pyranol (If)
6,8 GC-Gew-% cis-Pyranol (Ie)

Die Destillationsausbeute bezüglich cis- und trans-Pyranol (Ie und If) betrug ca. 90 %.

### Beispiel 4:

Die erste Kolonne war identisch aufgebaut wie die im Beispiel 3 eingesetzte und wurde identisch betrieben.
Die zweite Laborkolonne wurde als Trennwandkolonne aufgebaut. Sie war aus 6 doppelmanteligen, innenverspiegelten und evakuierten Glasschüssen mit 43 mm Innendurchmesser aufgebaut. Die Kolonnenschüsse 2 und 3 (von unten gezählt) mit einer Gesamtlänge von 232 cm waren mit einer fest eingeschmolzenen Trennwand aus etwa 1 mm starkem Glas versehen. Im Bereich der Trennwand ist die Kolonne mit 0,6 m Sulzer DX-Packung auf der Zulaufseite und auf der Abnahmeseite ausgerüstet. Oberhalb der Trennwand wurden zwei 60 cm und ein 30 cm langer Glassschuss eingesetzt, die mit insgesamt 147 cm Sulzer CY- Packungen ausgerüstet wurden. Unterhalb der Trennwand wurde ein 100 cm langer Glassschuss eingesetzt, der mit 64 cm Sulzer CY- Packungen ausgerüstet wurde. Die Trennleistung im Trennwandbereich betrug ca. 11 theoretische Stufen. Die Gesamtzahl der theoretischen Stufen inklusive des Trennwandbereiches betrug ca. 45.
Die Beheizung der Kolonne erfolgte mit einem ölbeheizten Laborumlaufdünnschichtverdampfer. Am Kopf der Kolonne wurden die Brüden mit einem Thermostat-gekühlten Glaskondensator niedergeschlagen.
Temperaturen an verschiedenen Höhen in der Kolonne sowie der Kopfdruck und der Druckverlust über die Kolonne wurden mittels eines Messwerterfassungssystems gemessen. Die Kolonne verfügte über Durchflussmessungen in den Zu- und Abläufen sowie eine Mengenmessung des Rücklaufs, die als Regelgröße für die Vorlauftemperatur des Ölthermostaten diente. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.
Der Zulauf wurde mit Hilfe eines ölbeheizten Glaswärmetauschers auf 80 °C aufgeheizt und auf Höhe der Mitte des Trennwandteiles zugegeben. Als Zulaufstrom wurde das Gemisch aus dem Sumpfablauf der ersten Kolonne verwendet. Der Zulaufmengenstrom betrug 398 g/h.
Die Kolonne wurde bei 10 mbar Kopfdruck und einem Rücklauf von 874 g/h betrieben. Dabei stellte sich ein Druckverlust von etwa 4,5 mbar ein. Am Kopf der Kolonne wurde eine Temperatur von 96°C und im Sumpf eine Temperatur von 120 °C (± 0,5 K) gemessen. Der Sumpfabzug wurde über eine Waagensteuerung fest auf 32 g/h (± 1 g/h) und die Destillatabnahme auf 46 g/h (± 1 g/h) eingestellt. Das Rücklaufverhältnis betrug somit etwa 19:1.
Die Flüssigkeit wurde oberhalb der Trennwand in einem Verhältnis von 1 : 2 (Zulauf- : Entnahmeteil) aufgeteilt. Auf der der Zugabeseite gegenüberliegenden Seite der Trennwand wurde auf gleicher Höhe wie der Zulaufstrom ein flüssiger Seitenabzug entnommen. Der Mengenstrom war fest auf 319 g/h eingestellt.

Das am Seitenabzug gewonnene Reinprodukt enthielt:
22,1 GC-Gew-% trans-Pyranol (If)
77,0 GC-Gew-% cis-Pyranol (Ie)

Der am Kopf der Kolonne abgezogene Kopfstrom enthielt:
24,3 GC-Gew-% Dihydropyran-Isomere (IVa - c)
0,40 GC-Gew-% Isoprenol
18,3 GC-Gew-% 1,3 Dioxan (Va)
45,6 GC-Gew-% trans-Pyranol (If)
4,2 GC-Gew-% cis-Pyranol (Ie)

Der am Sumpf der Kolonne abgezogene Strom enthielt:
2,0 GC-Flächen-% trans-Pyranol (If)
34,0 GC-Flächen-% cis-Pyranol (Ie)

Die Destillationsausbeute bezüglich cis- und trans-Pyranol (Ie und If) betrug ca. 90 %.

## Patentansprüche

1. Verfahren zur Herstellung von 2-substituierten 4-Hydroxy-4-methyl-tetrahydropyranen der Formel (I) 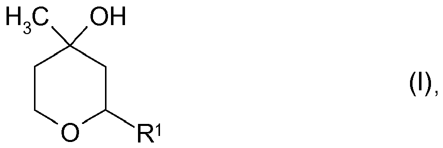 wobei der Rest
R¹ einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen bedeutet,
umfassend die Umsetzung von 3-Methylbut-3-en-1-ol der Formel (II) 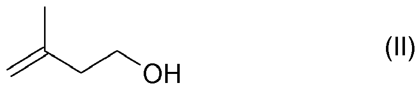 mit einem Aldehyd der Formel (III)
R¹-CHO (III),
wobei der Rest R¹ die gleiche Bedeutung wie in Formel (I) hat und
wobei man die Umsetzung in Gegenwart von Wasser und in Gegenwart eines stark sauren Kationenaustauschers durchführt, und anschließend die destillative Abtrennung in einer Trennwandkolonne oder in einer Zusammenschaltung von mindestens zwei Destillationskolonnen in Form einer thermischen Kopplung und einer oder mehreren Seitenabzugsstellen bei einem absoluten Betriebsdruck bis 500 mbar durchführt.

2. Verfahren nach Anspruch 1, **dadurch gekennzeichnet, dass** der Rest
R¹ einen geradkettigen oder verzweigten Alkyl- oder Alkenylrest mit 1 bis 12 Kohlenstoffatomen, oder einen gegebenenfalls Alkyl- und/oder Alkoxy-substituierten Arylrest mit insgesamt 6 bis 12 Kohlenstoffatomen
bedeutet.

3. Verfahren nach Anspruch 1 oder 2, **dadurch gekennzeichnet, dass** der Rest R¹ Isobutyl bedeutet.

4. Verfahren nach Anspruch 1 oder 2, **dadurch gekennzeichnet, dass** der Rest R¹ Phenyl bedeutet.

5. Verfahren nach einem der Ansprüche 1 bis 4, **dadurch gekennzeichnet, dass** man Isoprenol und den Aldehyd der Formel (III) in einem molaren Verhältnis von 0,7 zu 1 bis 2 zu 1 einsetzt.

6. Verfahren nach Anspruch 5, **dadurch gekennzeichnet, dass** man Isoprenol und den Aldehyd der Formel (III) in einem molaren Verhältnis von 1 zu 1 bis 1,5 zu 1 einsetzt.

7. Verfahren nach einem der Ansprüche 1 bis 6, **dadurch gekennzeichnet, dass** man die Umsetzung in Gegenwart einer mindestens äquimolaren Menge Wasser durchführt, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

8. Verfahren nach einem der Ansprüche 1 bis 7, **dadurch gekennzeichnet, dass** man einen stark sauren, Sulfonsäuregruppen umfassenden Kationenaustauscher einsetzt.

9. Verfahren nach einem der Ansprüche 1 bis 8, **dadurch gekennzeichnet, dass** man mindestens einen stark sauren Kationenaustauscher in der H(+)-Form einsetzt, wobei der Ionenaustauscher ein Sulfonsäuregruppen aufweisendes Polymergerüst enthält und entweder gelförmig ist oder makroporöse Harze enthält.

10. Verfahren gemäss einem der Ansprüche 1 bis 9, **dadurch gekennzeichnet, dass** der Ionenaustauscher auf einem Polystyrolgerüst mit Sulfonsäuregruppen oder auf einem perfluorierten Ionenaustauscherharz mit Sulfonsäuregruppen basiert.

11. Verfahren nach einem der Ansprüche 1 bis 10, **dadurch gekennzeichnet, dass** man die Umsetzung ohne Zusatz eines organischen Lösungsmittels durchführt.

12. Verfahren nach einem der Ansprüche 1 bis 11, **dadurch gekennzeichnet, dass** man die Umsetzung bei einer Temperatur im Bereich von 20 bis 60°C durchführt.

13. Verfahren nach einem der Ansprüche 1 bis 12, **dadurch gekennzeichnet, dass** man die Umsetzung kontinuierlich durchführt.

14. Verfahren nach Anspruch 13, umfassend die Schritte
a. Bereitstellen eines den gewählten stark sauren Kationenaustauscher enthaltenden Durchflussreaktors;
b. kontinuierliches Eintragen von Isoprenol, dem Aldehyd der Formel (III) sowie Wasser in den Durchflussreaktor;
c. kontinuierliches Inkontaktbringen von Isoprenol, dem Aldehyd der Formel (III) sowie Wasser mit dem stark sauren Kationenaustauscher im Durchflussreaktor unter Erhalt eines die gewünschten 2-substituierten 4-Hydroxy-4-methyl-tetrahydropyrane umfassenden Reaktionsgemisches und
d. kontinuierliches Austragen des Reaktionsgemisches aus dem Durchflussreaktor, und
e. Isolierung bzw. destillative Abtrennung in einer Trennwandkolonne oder in einer Zusammenschaltung von zwei Destillationskolonnen in Form einer thermischen Kopplung und einer oder mehrerer Seitenabzugsstellen bei einem absoluten Betriebsdruck bis 500 mbar, vorzugsweise von 3 bis 200 mbar.

15. Verfahren nach einem der Ansprüche 1 bis 14 zur Herstellung von 2-substituierten 4-Hydroxy-4-methyl-tetrahydropyranen in Form von Gemischen der cis-Diastereomeren der Formel (Ic) 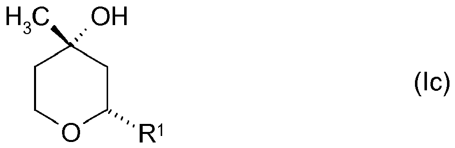 und der trans-Diastereomeren der Formel (Id) 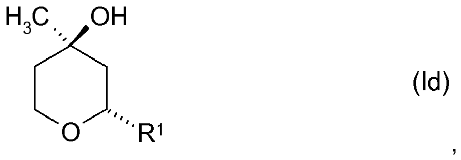 wobei das Diastereomerenverhältnis des cis-Diasteromeren der Formel (Ic) zum trans-Diastereomeren der Formel (Id) 65 zu 35 bis 95 zu 5 beträgt, und R¹ die in einem der Ansprüche 1, 2, 3 und 4 angegebene Bedeutung hat.

16. Verfahren gemäss Anspruch 1 zur kontinuierlichen Isolierung zweier stereoisomerer Alkohole der Formel (I) aus einem Rohgemisch durch Rektifikation, wobei man das Rohgemisch seitlich in eine Zulaufkolonne einführt, wenigstens eine mit der Zulaufkolonne gekoppelte Abzugskolonne vorsieht und aus der/den Abzugskolonne(n) einen ersten Alkohol der Formel (I) und einen zweiten Alkohol der Formel (I) abzieht, wobei man die Zulaufkolonne und die Abzugskolonnen so koppelt, dass zumindest im Bereich des Abzugs der Alkohole keine Quervermischung von Brüden und Kondensat zwischen Zulaufkolonne und Abzugskolonne(n) erfolgt.

17. Verfahren gemäss Anspruch 16, wobei man
a) das Rohgemisch in eine Zulaufkolonne mit oberhalb der Zulaufstelle gelegenem Verstärkungsteil und unterhalb der Zulaufstelle gelegenem Abtriebsteil einführt,
b) eine mit dem oberen Ende des Verstärkungsteilskommunizierende obere Vereinigungskolonne mit Kondensator am Kolonnenkopf und eine mit dem unteren Ende des Abtriebsteils kommunizierende untere Vereinigungskolonne mit Aufheizer am Sumpf vorsieht,
c) eine mit der oberen Vereinigungskolonne und der unteren Vereinigungskolonne kommunizierende Abzugskolonne vorsieht, die zwei in der Längsrichtung der Abzugskolonne beabstandet zueinander angeordnete Seitenabzüge aufweist,
d) den ersten Alkohol am oberen Seitenabzug und den zweiten Alkohol am unteren Seitenabzug abzieht, und
e) am Kopf oder im oberen Bereich der oberen Vereinigungskolonne Leichtsieder und im Sumpf der unteren Vereinigungskolonne Schwersieder abzieht.

18. Verfahren gemäss Anspruch 17, wobei die Zulaufkolonne und die Abzugskolonne als beidseitig zu je einem Vereinigungsraum offene Teilkammer ausgebildet sind, die sich über einen Abschnitt der Längsausdehnung einer Destillationskolonne erstrecken, und durch eine Trennwand voneinander getrennt sind.

19. Verfahren gemäss Anspruch 18, wobei man den ersten Alkohol und den zweiten Alkohol in flüssiger Form an den Seitenabzügen der Trennwandkolonne abzieht.

20. Verfahren gemäss einem der Ansprüche 17 bis 19, wobei die Zulaufkolonne und/oder Abzugskolonne ganz oder in Teilbereichen mit geordneten Packungen oder Füllkörpern versehen sind.

21. Verfahren gemäss einem der Ansprüche 17 bis 20, wobei die Trennwand zumindest in einem Teilbereich wärmeisolierend ausgebildet ist.

22. Verfahren gemäss einem der Ansprüche 17 bis 21, wobei man das Rohgemisch teilweise oder vollständig dampfförmig in die Zulaufkolonne einführt.

## Claims

1. A process for the preparation of 2-substituted 4-hydroxy-4-methyltetrahydropyrans of the formula (I) 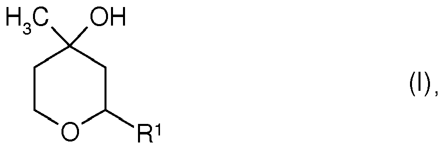 where the radical
R¹ is a straight-chain or branched alkyl or alkenyl radical having 1 to 12 carbon atoms, an optionally alkyl-substituted cycloalkyl radical having in total 3 to 12 carbon atoms or an optionally alkyl- and/or alkoxy-substituted aryl radical having in total 6 to 12 carbon atoms,
comprising the reaction of 3-methylbut-3-en-1-ol of the formula (II) 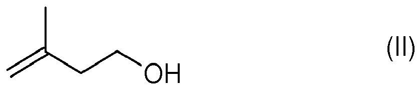 with an aldehyde of the formula (III)
R¹-CHO (III),
where the radical R¹ has the same meaning as in formula (I) and
where the reaction is carried out in the presence of water and in the presence of a strongly acidic cation exchanger, and then the distillative separation is carried out in a dividing wall column or in an interconnection of at least two distillation columns in the form of a thermal coupling and one or more side take-off points at an absolute operating pressure of up to 500 mbar.

2. The process according to claim 1, wherein the radical
R¹ is a straight-chain or branched alkyl or alkenyl radical having 1 to 12 carbon atoms, or an optionally alkyl- and/or alkoxy-substituted aryl radical having in total 6 to 12 carbon atoms.

3. The process according to claim 1 or 2, wherein the radical R¹ is isobutyl.

4. The process according to claim 1 or 2, wherein the radical R¹ is phenyl.

5. The process according to any one of claims 1 to 4, wherein isoprenol and the aldehyde of the formula (III) are used in a molar ratio of from 0.7:1 to 2:1.

6. The process according to claim 5, wherein isoprenol and the aldehyde of the formula (III) are used in a molar ratio of from 1:1 to 1.5:1.

7. The process according to any one of claims 1 to 6, wherein the reaction is carried out in the presence of an at least equimolar amount of water, where the amount of water refers to the amount of the starting material isoprenol used, optionally in deficit, or to the aldehyde of the formula (III), or, in the case of the equimolar reaction of the two starting materials, to the quantitative amount of one of the two.

8. The process according to any one of claims 1 to 7, wherein a strongly acidic cation exchanger comprising sulfonic acid groups is used.

9. The process according to any one of claims 1 to 8, wherein at least one strongly acidic cation exchanger is used in the H(+) form, where the ion exchanger comprises a polymer backbone having sulfonic acid groups and is either gel-like or comprises macroporous resins.

10. The process according to any one of claims 1 to 9, wherein the ion exchanger is based on a polystyrene backbone with sulfonic acid groups or on a perfluorinated ion exchange resin with sulfonic acid groups.

11. The process according to any one of claims 1 to 10, wherein the reaction is carried out without the addition of an organic solvent.

12. The process according to any one of claims 1 to 11, wherein the reaction is carried out at a temperature in the range from 20 to 60°C.

13. The process according to any one of claims 1 to 12, wherein the reaction is carried out continuously.

14. The process according to claim 13, comprising the steps
a. providing a flow reactor comprising the selected strongly acidic cation exchanger;
b. continuously introducing isoprenol, the aldehyde of the formula (III) and water into the flow reactor;
c. continuously bringing isoprenol, the aldehyde of the formula (III) and water into contact with the strongly acidic cation exchanger in the flow reactor to give a reaction mixture comprising the desired 2-substituted 4-hydroxy-4-methyltetrahydropyrans;
d. continuously discharging the reaction mixture from the flow reactor, and
e. isolating and/or distillatively separating in a dividing wall column or in an interconnection of two distillation columns in the form of a thermal coupling and one or more side take-off points at an absolute operating pressure of up to 500 mbar, preferably from 3 to 200 mbar.

15. The process according to any one of claims 1 to 14 for the preparation of 2-substituted 4-hydroxy-4-methyltetrahydropyrans in the form of mixtures of the cis-diastereomers of the formula (Ic) 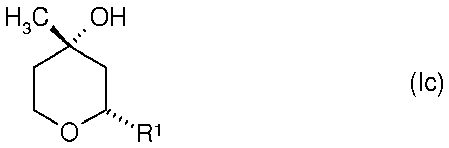 and of the trans-diastereomers of the formula (Id) 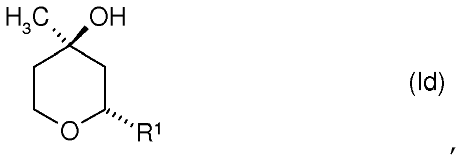 where the diastereomer ratio of the cis-diasteromer of the formula (Ic) to the trans-diastereomer of the formula (Id) is 65:35 to 95:5, and R¹ has the meaning given in any one of claims 1, 2, 3 and 4.

16. The process according to claim 1 for the continuous isolation of two stereoisomeric alcohols of the formula (I) from a crude mixture by rectification, where the crude mixture is introduced into a feed column at the side, at least one take-off column coupled to the feed column is provided and a first alcohol of the formula (I) and a second alcohol of the formula (I) are drawn off from the take-off column(s), where the feed column and the take-off columns are coupled such that at least in the region of the take-off of the alcohols, no crossmixing of vapors and condensate between feed column and take-off column(s) takes place.

17. The process according to claim 16, where
a) the crude mixture is introduced into a feed column with rectifying section positioned above the feed point and stripping section positioned below the feed point,
b) an upper combining column communicating with the upper end of the rectifying section and with a condenser at the top of the column, and a lower combining column communicating with the lower end of the stripping section and having a heater at the bottom are provided,
c) a take-off column communicating with the upper combining column and the lower combining column is provided which has two side take-offs arranged in the longitudinal direction of the take-off column at a distance from one another,
d) the first alcohol is drawn off at the upper side take-off and the second alcohol is drawn off at the lower side take-off, and
e) low-boiling components are drawn off at the top or in the upper region of the upper combining column, and high-boiling components are drawn off in the bottom of the lower combining column.

18. The process according to claim 17, where the feed column and the take-off column are designed as part chambers open on both sides to in each case one combining space which extend over a section of the longitudinal expansion of a distillation column, and are separated from one another by a dividing wall.

19. The process according to claim 18, where the first alcohol and the second alcohol are drawn off in liquid form at the side take-offs of the dividing wall column.

20. The process according to any one of claims 17 to 19, where the feed column and/or take-off column are provided entirely or in sections with structured packings or random packings.

21. The process according to any one of claims 17 to 20, where the dividing wall is designed to be thermally insulating at least in one section.

22. The process according to any one of claims 17 to 21, where the crude mixture is introduced into the feed column partially or completely in vapor form.

## Revendications

1. Procédé pour la préparation de 4-hydroxy-4-méthyltétrahydropyranes substitués en 2ème position de formule (I) 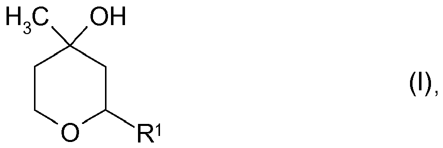 dans laquelle le radical
R¹ signifie un radical alkyle ou alcényle linéaire ou ramifié comprenant 1 à 12 atomes de carbone, un radical cycloalkyle le cas échéant substitué par alkyle présentant au total 3 à 12 atomes de carbone ou un radical aryle le cas échéant substitué par alkyle et/ou alcoxy présentant au total 6 à 12 atomes de carbone,
comprenant la transformation de 3-méthylbut-3-én-1-ol de formule (II) 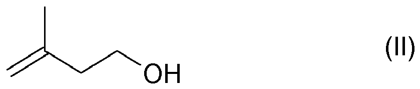 avec un aldéhyde de formule (III)
R¹-CHO (III),
le radical R¹ ayant la même signification que dans la formule (I) et dans lequel on réalise la transformation en présence d'eau et en présence d'un échangeur de cations fortement acide, puis on réalise la séparation par distillation dans une colonne à paroi de séparation ou dans une commutation d'au moins deux colonnes de distillation sous forme d'un couplage thermique et présentant un ou plusieurs sites de soutirage latéraux à une pression d'exploitation absolue jusqu'à 500 mbars.

2. Procédé selon la revendication 1, **caractérisé en ce que** le radical
R¹ signifie un radical alkyle ou alcényle linéaire ou ramifié comprenant 1 à 12 atomes de carbone ou un radical aryle le cas échéant substitué par alkyle et/ou alcoxy comprenant au total 6 à 12 atomes de carbone.

3. Procédé selon la revendication 1 ou 2, **caractérisé en ce que** le radical R¹ signifie isobutyle.

4. Procédé selon la revendication 1 ou 2, **caractérisé en ce que** le radical R¹ signifie phényle.

5. Procédé selon l'une quelconque des revendications 1 à 4, **caractérisé en ce qu'**on utilise l'isoprénol et l'aldéhyde de formule (III) dans un rapport molaire de 0,7:1 à 2:1.

6. Procédé selon la revendication 5, **caractérisé en ce qu'**on utilise l'isoprénol et l'aldéhyde de formule (III) dans un rapport molaire de 1:1 à 1,5:1.

7. Procédé selon l'une quelconque des revendications 1 à 6, **caractérisé en ce qu'**on réalise la transformation en présence d'une quantité au moins équimolaire d'eau, la quantité d'eau se rapportant à la quantité de la substance de départ, le cas échéant utilisée en une quantité inférieure à la quantité stoechiométrique, l'isoprénol ou l'aldéhyde de formule (III) ou, dans le cas d'une transformation équimolaire des deux substances, à la quantité d'une des deux substances.

8. Procédé selon l'une quelconque des revendications 1 à 7, **caractérisé en ce qu'**on utilise un échangeur de cations fortement acide, comprenant des groupes acide sulfonique.

9. Procédé selon l'une quelconque des revendications 1 à 8, **caractérisé en ce qu'**on utilise au moins un échangeur de cations fortement acide sous forme H(+), l'échangeur d'ions contenant une structure polymère présentant des groupes acide sulfonique et étant sous forme de gel ou contenant des résines macroporeuses.

10. Procédé selon l'une quelconque des revendications 1 à 9, **caractérisé en ce que** l'échangeur d'ions est à base d'une structure de polystyrène présentant des groupes acide sulfonique ou à base d'une résine échangeuse d'ions perfluorée présentant des groupes acide sulfonique.

11. Procédé selon l'une quelconque des revendications 1 à 10, **caractérisé en ce qu'**on réalise la transformation sans addition d'un solvant organique.

12. Procédé selon l'une quelconque des revendications 1 à 11, **caractérisé en ce qu'**on réalise la transformation à une température dans la plage de 20 à 60°C.

13. Procédé selon l'une quelconque des revendications 1 à 12, **caractérisé en ce qu'**on réalise la transformation en continu.

14. Procédé selon la revendication 13, comprenant les étapes
a. mise à disposition d'un réacteur à écoulement contenant l'échangeur de cations fortement acide choisi ;
b. introduction continue d'isoprénol, de l'aldéhyde de formule (III) ainsi que d'eau dans le réacteur à écoulement ;
c. mise en contact continue d'isoprénol, de l'aldéhyde de formule (III) ainsi que d'eau avec l'échangeur de cations fortement acide dans le réacteur à écoulement avec obtention d'un mélange réactionnel contenant le 4-hydroxy-4-méthyltétrahydropyrane substitué en 2ème position souhaité ;
d. évacuation continue du mélange réactionnel du réacteur à écoulement et
e. isolement ou séparation par distillation dans une colonne à paroi de séparation ou dans une commutation d'au moins deux colonnes de distillation sous forme d'un couplage thermique et présentant un ou plusieurs sites de soutirage latéraux à une pression d'exploitation absolue jusqu'à 500 mbars, de préférence de 3 à 200 mbars.

15. Procédé selon l'une quelconque des revendications 1 à 14 pour la préparation de 4-hydroxy-4-méthyltétrahydropyranes substitués en 2ème position sous forme de mélanges des diastéréo-isomères cis de formule (Ic) 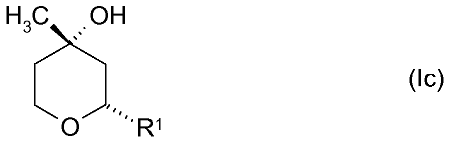 et des diastéréo-isomères trans de formule (Id) 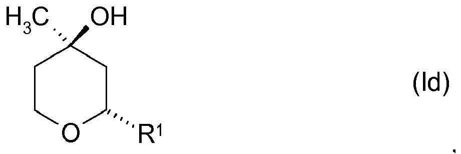 le rapport des diastéréo-isomères cis de formule (Ic) aux diastéréo-isomères trans de formule (Id) valant 65:35 à 95:5, et R¹ ayant la signification indiquée dans l'une quelconque des revendications 1, 2, 3 et 4.

16. Procédé selon la revendication 1 pour l'isolement continu de deux alcools stéréo-isomères de formule (I) à partir d'un mélange brut par rectification, le mélange brut étant introduit latéralement dans une colonne d'alimentation, au moins une colonne de soutirage couplée à la colonne d'alimentation étant prévue et un premier alcool de formule (I) et un deuxième alcool de formule (I) étant soutirés de la/des colonne(s) de soutirage, la colonne d'alimentation et les colonnes de soutirage étant couplées de manière telle qu'il ne se produit pas de mélange transversal des vapeurs et du condensat, au moins au niveau du soutirage des alcools, entre la colonne d'alimentation et la/les colonne(s) de soutirage.

17. Procédé selon la revendication 16, dans lequel
a) on introduit le mélange brut dans une colonne d'alimentation présentant une partie d'enrichissement située au-dessus du site d'alimentation et une partie d'épuisement située sous le site d'alimentation,
b) on prévoit une colonne de rassemblement supérieure communiquant avec l'extrémité supérieure de la partie d'enrichissement avec un condenseur en tête de colonne et une colonne de rassemblement inférieure communiquant avec l'extrémité inférieure de la partie d'épuisement présentant un chauffage dans le fond,
c) on prévoit une colonne de soutirage communiquant avec la colonne de rassemblement supérieure et la colonne de rassemblement inférieure qui présente deux soutirages latéraux disposés à une certaine distance l'un de l'autre dans la direction longitudinale de la colonne de soutirage,
d) on soutire le premier alcool au niveau du soutirage latéral supérieur et le deuxième alcool au niveau du soutirage latéral inférieur, et
e) on soutire la fraction légère en tête ou dans la partie supérieure de la colonne de rassemblement supérieure et la fraction lourde dans le fond de la colonne de rassemblement inférieure.

18. Procédé selon la revendication 17, la colonne d'alimentation et la colonne de soutirage étant réalisées sous forme de chambres partielles ouvertes des deux côtés vers à chaque fois une chambre de rassemblement, qui s'étendent sur une section de l'étendue longitudinale d'une colonne de distillation et qui sont séparées l'une de l'autre par une paroi latérale.

19. Procédé selon la revendication 18, dans lequel on soutire le premier alcool et le deuxième alcool sous forme liquide au niveau des soutirages latéraux de la colonne à paroi de séparation.

20. Procédé selon l'une quelconque des revendications 17 à 19, la colonne d'alimentation et/ou la colonne de soutirage étant pourvues, complètement ou dans des zones partielles, de garnissages ordonnés ou de corps de remplissage.

21. Procédé selon l'une quelconque des revendications 17 à 20, la paroi de séparation étant réalisée de manière thermiquement isolée sur au moins une zone partielle.

22. Procédé selon l'une quelconque des revendications 17 à 21, le mélange brut étant introduit partiellement ou complètement sous forme de vapeur dans la colonne d'alimentation.
